# NEW EUROPEAN PATENT SPECIFICATION

(11) **EP 0 370 674 B2**
(45) Date of publication and mention of the opposition decision: **15.12.1999**
(45) Mention of the grant of the patent: 10.02.1993
(21) Application number: 89311745.7
(22) Date of filing: 14.11.1989
(51) Int. Cl.: F16B 25/10, F16B 25/00

(54) **Drill screw**
Bohrschraube
Vis perceuse

(30) Priority: 25.11.1988 JP 29733488
(43) Date of publication of application: 30.05.1990
(73) Proprietor: Yugen Kaisha Shinjoseisakusho, Nishinariku Osaka (JP)
(72) Inventor: Shinjo, Katsumi, Nishinariku Osaka (JP)
(74) Representative: Price, Paul Anthony King

(56) References cited:
- WO-A-88/05991
- CH-A- 608 575
- DE-A- 2 019 040
- DE-A- 2 555 647
- DE-A- 3 235 447
- US-A- 1 004 795
- US-A- 4 736 481
- Taschenbuch Umformtechnik, Heinz Tschätsch, Carl Hanser Verlag München Wien, "Verfahren Maschinen Werkzeuge", 1977, page 68
- DIN 7970, November 1984, page 379, "Gewinde und Schraubenden für Blechschrauben"

## Description

The invention relates to a drill screw.

A drill screw, having a drill bit formed at a tip end of a screw which also has a driving head, can be advantageous in that forming holes in workpieces to be fastened, tapping of the holes and fastening of the workpieces together by means of the screw can be carried out in one continuous operation. Therefore, such drill screws have become increasingly popular. As new and various uses have been found for such drill screws, there have arisen problems in some cases. For example, the drill bit and a superfluous portion of threaded stem adjoining the drill bit may protrude from a rear surface of a structure after components thereof have been fastened together by the drill screw, thereby spoiling the appearance of the structure and further being likely to inflict injury on a person.

It is known, from DE-A-2555647, to provide a drill screw comprising a first shank of diameter D made of an austenitic stainless steel not susceptible of hardening; a driving head extending from an end of the first shank and engageable with a screw driving tool; a second shank made of a low carbon steel susceptible of hardening, the second shank being secured to the first shank; a drill bit; and a continuous thread extending around the outer cylindrical periphery of the first shank.

A disadvantage of this arrangement is that it is not easily possible to detach the second shank and the drill bit from the first shank once the drilling action has been completed.

It has also been proposed, in DE-A-2019040, to provide a drill screw having a break zone so that the drill bit portion of the drill screw can readily be detached from the remainder thereof.

However, DE-A-2019040 does not address the problem of providing such a break zone together with an effective screw thread in a composite drill screw of the kind disclosed in DE-A-2555647. Therefore, the drilling and screwing performance of the drill screw of DE-A-2019040 is inferior to that of the drill screw of DE-A-2555647.

It should be noted that a proposal to form a circumferential groove at a position where a screw is to be broken to permit removal of a protruding superfluous portion would not, alone, be effective. Such a common practice cannot be applied to drill screws since they are hardened so as to have sufficient hardness for machining or cutting by their drill bits and also for tapping by their threaded stems adjoining the drill bits. The thus hardened surface would make it difficult to break the screw at such a grooved portion thereof.

Fig. 4 of WO-A-88/05991 discloses a drill screw in which the continuous thread extends from the first shank to the second shank. The first shank is welded to the second shank across the full width of the root of the continuous thread by a stored energy welding process. Fig. 17 of WO-A-88/05991 discloses a drill rivet having a drill bit welded to the tip of a rivet by a stored energy welding process. The drill bit may be broken off at the welded joint after the drilling operation.

According to a first aspect of the invention there is provided a drill screw comprising: a first shank of diameter D made of metal unsusceptible of hardening; a driving head extending from an end of the first shank and engageable with a screw driving tool; a flat surface formed at the other end of the first shank extending perpendicular to the longitudinal axis of the first shank; a second shank made of a metal susceptible of hardening and of the same diameter as the first shank, the second shank being secured to the flat surface to provide a zone at the other end of the first shank; a drill bit formed by cold forging at the other end of the second shank; and a continuous thread extending on and around the outer cylindrical periphery of the first shank, wherein the continuous thread is formed by thread rolling so as to extend additionally on and around the outer cylindrical periphery of the second shank, the portion extending around the second shank extending for at least one screw thread pitch and the portion extending from the zone to the drill bit being hardened; characterised in that:
(i) the second shank is secured to the flat surface by projection welding;
(ii) the zone is a break zone at which the drill screw can be broken; and
(iii) within the root of the continuous thread, the first and second shanks are only partially joined, at the break zone, and at the inner part of the shanks.

Thus an end portion of the threaded stem protruding from a back surface of a structure having components fastened by means of the drill screw can easily be broken off and removed.

A drill screw in accordance with the invention can have a structure which enables easier and neater breaking or snapping and subsequent removal of the drill bit and superfluous threaded portion so that it does not project from a rear surface of a fastened object.

According to a second aspect of the invention, there is provided a method of manufacturing a drill screw as defined above, comprising the step of projection welding to a surface formed at one end of a first shank an end surface of a second shank including a welding lug projecting therefrom, whereby the ends of the shanks are secured in an abutting relationship with a break zone formed therebetween.

The invention is diagrammatically illustrated by way of example in the accompanying drawings, in which:-
Figure 1 is a front elevation showing a first shank of a drill screw according to the invention;
Figure 2 is a front elevation showing a second shank of a drill screw according to the invention;
Figure 3 is a front elevation of a blank to form a drill screw according to the invention;
Figure 4 is a front elevation illustrating a process by which a drill bit is formed on the blank of Figure 3; and
Figure 5 is a front elevation illustrating a complete drill screw according to the invention.

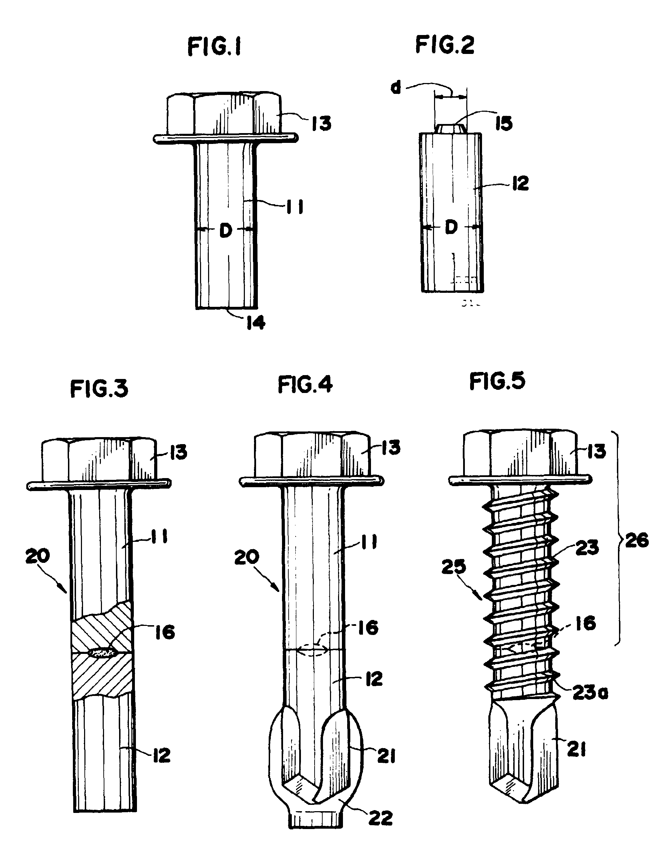

Referring to the drawings, Figure 1 shows a first shank 11 of a drill screw and Figure 2 shows a second shank 12.

The first shank 11 is made of austenitic stainless steel, which is a metal unsusceptible of carburizing-and-quenching, and is of a predetermined diameter "D" suited to thread-rolling formation of a screw thread as described hereinafter. The first shank 11 has at one end a driving head 13 which is formed by a header-machining technique to be of a shape engageable with a screw driving tool, whereas the other end of the first shank 11 is machined to a flat surface 14.

The second shank 12 is made of a low-carbon steel which is susceptible of carburizing-and-quenching, the second shank 12 being of the same diameter "D" as the first shank 11, and having a welding lug to be of a shape engageable with a screw driving tool, whereas the other end of the first shank 11 is machined to a flat surface 14.

The second shank 12 is made of a metal of another kind, for instance, a low-carbon steel which is susceptible of carburizing-and-quenching, the second shank 12 being of the same diameter "D" as the first shank 11, and having a welding lug 15 integral with and projecting from a middle zone of an end surface of one end of the second shank 12. The welding lug 15 which will form a break zone 16 as described later has a maximum diameter "d" which is predetermined to be included within a range from 0.5D to 0.8D. Such a dimension is selected here in view of the fact that a drilling and tapping torque necessary for the drill bit and threaded stem portion adjoining it to dig a hole in a workpiece and subsequently to tap the hole is generally low compared with a fastening torque for tightening a main portion of the threaded stem. The value of the drilling and tapping torque is equal to, for instance about one third (1/3) to two thirds (2/3) of the fastening torque. Thus, the comparatively small diameter "d" of the welding lug 15 can easily withstand a force imparted thereto when drilling and tapping are simultaneously carried out by means of the drill bit and the threaded portion adjoining it, while at the same time allowing these portions to be broken off quite easily from the main threaded portion.

Figures 3 to 5 illustrate steps in the manufacture of the drill screw from the first shank 11 and the second shank 12.

At first, the flat end surface 14 of the first shank 11 is caused by a projection-welding method, as shown in Figure 3, fixedly to adjoin the welding lug 15 of the second shank 12 in order that the break zone 16 is formed at bonded portion between the 11, 12 shanks whereby a composite blank 20 is produced.

Next, a drill bit 21 is formed by cold forging of the second shank 12 in the blank 20, as shown in Figure 4. A machining mould or die used for cold forging of the drill bit 21 is not shown because it is well known in the industry. Such machining will produce a burr 22 along and projecting from an outer periphery of the drill bit 21.

Subsequently, a continuous screw thread 23 and 23a is formed as shown in Figure 5 by a thread-rolling method employing a known rolling die (not shown) so as to extend around the outer cylindrical peripheries of the first shank 11 and the second shank 12. The burr 22 referred to above is removed when the thread-rolling is carried out to form the thread, thereby finishing up the drill bit 21. The threaded portion 23a formed by thread-rolling on the second shank 12 desirably extends up to close proximity with the drill bit 21 in a state that it comprises at least one screw thread pitch which functions as a tapping screw when tapping is effected as described below.

A drill screw 25 which has been machined in a manner described above is then subjected to a carburizing-and quenching treatment This treatment causes carburizing of, i.e., diffusion of carbon into, surface layers of the drill bit 21 and the threaded portion 23a which are formed on the second shank 12, thereby to impart a predetermined hardness to the surface layers. However, the threaded portion 23 formed on the first shank 11 is not carburized due to the unsusceptible nature of the metal of which the first shank is made. The break zone 16 located at the bonded or fixedly adjoining portions between the first and second shanks 11,12 also is not hardened by carburizing-and quenching since said zone is at an inner part of the shanks.

The drill screw 25 which is manufactured to obtain the above described structure can be used to fasten one object to another (not shown). After a fastening operation is completed, the drill bit 21 and the threaded portion 23a which are likely to protrude from a rear surface of said another object are easily broken at the break zone 16 and separated from a remaining main party 26. The remaining main part 26 comprising the first shank 11 is highly corrosion-resistant by virtue of austenitic stainless steel of which the first shank 11 is made.

If it is deemed appropriate for material of the second shank 12 and for the character of the fastened objects, any other usual hardening or quenching treatment may be utilized in place of the exemplified carburising-and-quenching in order to harden to a satisfactory degree the surfaces of the drill bit 21 and the threaded portion 23a which are formed from the second shank 12.

## Claims

1. A drill screw comprising:
a first shank (11) of diameter D made of metal unsusceptible of hardening;
a driving head (13) extending from an end of the first shank (11) and engageable with a screw driving tool;
a flat surface formed at the other end of the first shank extending perpendicular to the longitudinal axis of the first shank;
a second shank (12) made of a metal susceptible of hardening and of the same diameter as the first shank, the second shank (12) being secured to the flat surface to provide a zone (16) at the other end of the first shank (11);
a drill bit (21) formed by cold forging at the other end of the second shank; and
a continuous thread (23 and 23a) extending on and around the outer cylindrical periphery of the first shank (11), wherein the continuous thread (23 and 23a) is formed by thread rolling so as to extend additionally on and around the outer cylindrical periphery of the second shank (12), the portion extending around the second shank extending for at least one screw thread pitch and the portion extending from the zone (16) to the drill bit (21) being hardened;
characterised in that:
(i) the second shank (12) is secured to the flat surface by projection welding;
(ii) the zone (16) is a break zone (16) at which the drill screw can be broken; and
(iii) within the root of the continuous thread (23 and 23a), the first and second shanks (11 and 12) are only partially joined, at the break zone (16), and at the inner part of the shanks (11 and 12).

2. A method of manufacturing a drill screw according to claim 1, characterised by the step of projection welding to a surface formed at one end of a first shank (11) an end surface of a second shank (12) including a welding lug (15) projecting therefrom, whereby the ends of the shanks (11, 12) are secured in an abutting relationship with a break zone (16) formed therebetween.

3. A method according to claim 2, characterised in that the diameter (d) of the welding lug is in the range of 0.5 - 0.8 times the diameter (D) of the first and second shanks.

## Patentansprüche

1. Bohrschraube mit
einem ersten Schaft (11) eines Durchmessers D aus einem für Härten unempfänglichen Metall,
einem Antriebskopf (13), der sich von einem Ende des ersten Schaftes (11) aus erstreckt und in Eingriff mit einem Schraubenantriebswerkzeug bringbar ist,
einer am anderen Ende des ersten Schaftes ausgebildeten glatten Oberfläche, die sich senkrecht zu der Längsachse des ersten Schaftes erstreckt,
einem zweiten Schaft (12) aus einem für Härtung empfänglichen Metall mit dem gleichen Durchmesser wie der erste Schaft, wobei der zweite Schaft (12) an der glatten Oberfläche befestigt ist, um eine Zone (16) an dem anderen Ende des ersten Schaftes (11) zu bekommen,
einer Bohrspitze (21), die am anderen Ende des zweiten Schaftes durch Kaltschmieden ausgebildet ist, und einem kontinuierlichen Gewinde (23 und 23a), das sich auf dem und um den zylindrischen Außenumfang des ersten Schaftes (11) erstreckt, worin das kontinuierliche Gewinde (23 und 23a) durch Gewindewalzen derart gebildet ist, daß es sich zusätzlich auf dem und um den zylindrischen Außenumfang des zweiten Schaftes (12) erstreckt, der sich um den zweiten Schaft erstreckende Abschnitt über wenigstens eine Schraubgewinde-Gangh öhe erstreckt und sich von der Zone (16) aus zu der Bohrspitze (21) erstreckende Abschnitt gehärtet ist,
**dadurch gekennzeichnet,** daß
i) der zweite Schaft (12) an der glatten Oberfläche durch Buckelschweißen befestigt ist,
ii) die Zone (16) eine Bruchzone (16) ist, an welcher die Bohrschraube gebrochen werden kann, und
iii) in dem Fuß des kontinuierlichen Gewindes (23 und 23a) der erste und zweite Schaft (11 und 12) nur teilweise bei der Bruchzone (16) und an dem Innenteil der Schäfte (11 und 12) verbunden sind.

2. Verfahren zur Herstellung einer Bohrschraube nach Anspruch 1, **gekennzeichnet durch** die Stufe eines Buckelschweißens einer Endoberfläche eines zweiten Schaftes (12) mit einem von ihr vorspringenden Schweißanguß (15) an eine an einem Ende eines ersten Schaftes (11) ausgebildete Oberfläche, wobei die Enden der Schäfte (11, 12) in einer aneinanderstoßenden Beziehung mit einer dazwischen gebildeten Bruchzone (16) befestigt werden.

3. Verfahren nach Anspruch 2, **dadurch gekennzeichnet**, daß der Durchmesser (d) des Schweißangusses im Bereich des 0,5- bis 0,8fachen des Durchmessers (D) des ersten und zweiten Schaftes ist.

## Revendications

1. Vis perceuse comprenant :
une première tige (11) de diamètre D faite d'un métal qui n'est pas susceptible d'être durci ;
une tête d'entraînement (13) s'étendant depuis une extrémité de la première tige (11) et pouvant être engagée avec un outil de vissage ;
une surface plate formée à l'autre extrémité de la première tige s'étendant perpendiculairement à l'axe longitudinal de la première tige ;
une seconde tige (12) faite d'un métal susceptible d'être durci et du même diamètre que la première tige, la seconde tige (12) étant fixée à la surface plate pour former une zone (16) à l'autre extrémité de la première tige (11) ;
un perçoir (21) formé par forgeage à froid à l'autre extrémité de la seconde tige ; et
un filet continu (23 et 23a) s'étendant sur, et autour de, la périphérie cylindrique externe de la première tige (11), le filet continu (23 et 23a) étant formé par laminage de manière à s'étendre en outre sur, et autour de, la périphérie cylindrique externe de la seconde tige (12), la portion s'étendant autour de la seconde tige (12) s'étendant sur au moins un pas de vis et la portion s'étendant de la zone (16) au perçoir (21) étant durcie ;
caractérisée en ce que :
(i) la seconde tige (12) est fixée à la surface plate par soudage à bossages ;
(ii) la zone (16) est une zone de rupture (16) à laquelle la vis perceuse peut être rompue ; et
(iii) au fond du filet continu (23 et 23a), la première tige et la seconde tige (11 et 12) ne sont que partiellement reliées, à la zone de rupture (16), et à la partie interne des tiges (11 et 12).

2. Procédé de fabrication d'une vis perceuse selon la revendication 1, caractérisé par la phase de soudage à bossages à une surface formée à une extrémité d'une première tige (11) d'une surface d'extrémité d'une seconde tige (12) comprenant une patte de soudage (15) en projection, de manière que les extrémités des tiges (11, 12) soient fixées bout à bout avec une zone de rupture (16) formée entre elles.

3. Procédé selon la revendication 2, caractérisé en ce que le diamètre (d) de la patte de soudage est dans la plage de 0,5 à 0,8 fois le diamètre (D) de la première tige et de la seconde tige.
